# EUROPEAN PATENT SPECIFICATION

(11) **EP 0 368 466 B1**
(45) Date of publication and mention of the grant of the patent: **29.11.1995**
(21) Application number: 89310044.6
(22) Date of filing: 02.10.1989
(51) Int. Cl.: A61K 39/395

(54) **Compositions and methods for the treatment and prevention of gram-negative bacterial infections**
Zusammensetzungen und Methoden zur Behandlung und Vorbeugung von Gram-Negativen-Infektionen
Compositions et méthodesde traitement et de prevention des infections à bacteries gram-negative

(30) Priority: 12.10.1988 US 257445
(43) Date of publication of application: 16.05.1990
(73) Proprietor: BAXTER INTERNATIONAL INC., Deerfield, IL 60015 (US)
(72) Inventor: Bubbers, J. Eric, Los Angeles, CA 90077 (US); Castaldi, David L., Chestnut Hill, MA 02167 (US); Mankarious, Samia, Cost Mesa, CA 92626 (US); Hooper, John A., Santa Ana, CA 92706 (US); Alpern, Melanie, Long Beach, CA 90808 (US)
(74) Representative: Bassett, Richard Simon

(56) References cited:
- EP-A- 0 163 493
- EP-A- 0 174 204
- EP-A- 0 183 876
- WO-A-86/07382
- GB-A- 2 185 266
- GB-A- 2 186 592
- GB-A- 2 192 185
- INFECTION & IMMUNITY, vol. 54, no. 1, October 1986, Washington, DC (US); J.E. PENNINGTON et al., pp. 239-244/
- CHEMICAL ABSTRACTS, vol. 101, no. 16, 15 October 1984, Columbus, OH (US); L.M. MUTHARIA et al., p. 532, no. 149444j/
- CHEMICAL ABSTRACTS, vol. 102, no. 1, 07 January 1985, Columbus, OH (US); M.J. NELLES et al., p. 521, no. 22552h/

## Description

### Field of the Invention

This invention relates to an intravenously injectable immune globulin product useful in the prophylaxis and therapy of diseases of Gram-negative bacterial origin. More specifically, this invention relates to such a product which comprises plasma-derived polyclonal immunoglobulins containing polyclonal antibodies against antigens of Gram-negative bacteria and monoclonal antibodies which can neutralize the endotoxin associated with the core lipopolysaccharides of Gram-negative bacteria.

### Background of the Invention

Gram-negative bacteria have become the leading cause of fatal bacterial infections in hospital patients. The mortality rate for patients suffering from Gram-negative bacteremia has approached forty percent in the past twenty years, despite aggressive antibiotic therapy. These bacteria are distinguished by a membrane that is relatively impermeable to drugs and by an endotoxin produced by all Gram-negative bacteria which remains lethally toxic even after the bacterial cells have been killed. The lethality of Gram-negative infections thus is due both to uncontrolled growth of the viable bacteria and to the release of endotoxin from the organisms which results in death by endotoxic shock.

The endotoxins, which are of great structural diversity and unique to Gram-negative bacteria, are associated with the lipopolysaccharide (LPS) component of the outer membrane of the bacteria. The LPS from different bacterial species usually are similarly comprised of three structural regions: the O-specific carbohydrate chain, the core, and the lipid A. The lipid A portion inserts into the outer membrane. Attached to the lipid A is the rough core region, which in turn is attached to the O-saccharide side chain. The O-side chain consists of a repeating structure of tri- to pentasaccharides containing the species and serotype specific determinants of Gram-negative cells. Substantial variation of the O antigens exists within a single bacterial species (e.g. Pseudomonas aeruginosa has at least 16 different possible O antigens). Less variation is shown in the covalently bound lipid A/core component, which represents the toxic principle of the endotoxin. Different bacterial species have chemically similar lipid A molecules which cause similar pathological effects in host tissues. It is for this reason that lipid A also is referred to as an endotoxin. The lipid A/core region can stimulate an antibody response if exposed by the removal of the O-specific antigen.

In view of the limited effectiveness of antibiotics in treating Gram-negative bacterial diseases, a number of efforts have been made to use antibodies against the lipopolysaccharides to provide immunity to Gram-negative organisms. Effective immunity involves antibody responses to both the variable O antigens as well as the conserved antigens on the lipid A molecule. Studies have shown that intravenous preparations of polyclonal IgG can be effective in decreasing the incidence of opportunistic nosocomial Gram-negative bacterial infections, especially in immunocompromised patients. Although the polyclonal immunoglobulin is effective in killing the bacterial organisms, such preparations have proved to be generally unsatisfactory in treating the endotoxic shock that frequently accompanies the infections. Typically, the antibody titer contained in the preparations is too low to display sufficient clinical potency to ameliorate the endotoxamia.

As a result, investigators have studied monoclonal antibodies against the bacterial endotoxins. To obtain a monoclonal antibody, a myeloma cell and an antibody-producing lymphocyte are fused together to produce a hybrid cell (a hybridoma) which combines the lymphocyte's ability to produce a specific (or monoclonal) antibody and the ability of the myeloma cell to propagate indefinitely. Although a monoclonal antibody is homogenous, it may not be monospecific. Monoclonal antibodies will react with different moieties that share an antigenic determinant or carry structurally-related determinants. Antigenic similarities have been demonstrated between the lipid A/core regions of E. coli and other species of bacteria. A shared antigenic determinant present in the LPS results in cross-reactive hybridoma-derived monoclonal antibodies (mAb) with broad in vivo and in vitro reactivities.

A number of researchers have reported the preparation of hybridomas which produce antibodies that recognize determinants in the lipid A portion of LPS. Although these results have looked promising, further research has shown that monoclonal antibody preparations when administered alone, generally are ineffective when administered to animals suffering from Gram-negative bacterial infections. Although the reasons for this are not fully understood, it is known that a low level exposure to endotoxin can protect against a higher dose of the same or other endotoxin or Gram-negative bacteria. Since endotoxin contamination is very common, it is possible that in some of the early studies the apparent animal protection against Gram-negative bacteria by the administration of monoclonal antibodies to endotoxin may have resulted from endotoxin contamination of the antibody preparation. (Chong, K.T. and Huston, M., 1987, J. Inf. Dis. 156:713-179.)

GB-A-2 185 266 discloses monoclonal antibodies cross-reactive with and cross-protective against P. aeruginosa serotypes. GB-A-2 186 592 discloses monoclonal antibodies against a variety of gram negative bacteria.

There remains a need for a method of providing a consistent and reproducible source of immunotherapeutic or prophylactic agents to combat Gram-negative bacteremia and endotoxemia. Accordingly, it is an object of the present invention to provide a composition and method for the treatment and prophylaxis of Gram-negative bacterial diseases which are the result of growth by the bacteria and/or endotoxins produced by those bacteria.

### Summary of the Invention

In accordance with the present invention, there are disclosed compositions, for use in the prophylaxis or treatment of infections caused by Gram-negative bacteria or endotoxic shock caused by the endotoxins the bacteria produce, which comprise a combination of human polyclonal immunoglobulins containing polyclonal antibodies against antigens of Gram-negative bacteria and hybridoma-derived monoclonal antibodies which are specific for epitopes common to a variety of Gram-negative bacteria contained in the lipid A moiety of the lipopolysaccharides. The invention also relates to a method of preventing or treating a disease of Gram-negative bacterial origin by administering to a patient in need of such prophylaxis or treatment an effective amount of a composition comprising a combination of polyclonal immunoglobulins containing antibodies against Gram-negative bacteria and hybridoma-derived monoclonal antibodies which can bind to those antigens common to the core/lipid A region of Gram-negative bacterial lipopolysaccharides.

### Detailed Description of the Invention

This invention relates to novel compositions for the prophylaxis or treatment of infectious or endotoxic agents of Gram-negative bacterial origin. Inasmuch as the morbidity and mortality associated with diseases of Gram-negative bacterial origin can be attributed both to the uncontrolled growth of the organism and to the effects of the lipopolysaccharide endotoxin it produces, effective treatment or prophylaxis much at least substantially eliminate the bacterial infection and neutralize the lipid A endotoxin. "Neutralize" refers to the reduction, and preferably the elimination, of the toxic effects of lipid A endotoxin, as evidenced, for example, by the protection against the lethal effects of endotoxin.

The novel compositions of this invention comprise a combination of two components. One component comprises a polyclonal immunoglobulin intravenous preparation which contains antibodies that recognize O-antigens specific for a variety of Gram-negative organisms and thus provide effective protection against those bacteria. The second component comprises monoclonal antibodies that recognize one antigen common to the core/lipid A region of a variety of Gram-negative bacterial lipopolysaccharides and so neutralize their endotoxin.

The compositions of this invention can be used to provide in vivo protection against a potentially lethal infection by one or more Gram-negative organisms and the endotoxin they produce. The polyclonal component of the composition protects against bacterial infection but generally does not afford adequate protection against endotoxin. The combination of polyclonal immunoglobulin and monoclonal anti-endotoxin antibody provides protection against death or illness by Gram-negative bacterial infection and by the endotoxin associated with that organism.

### Preparation of Monoclonal Antibodies

To obtain an anti-lipid A monoclonal antibody, a cell line is established that will grow in tissue culture and secrete an antibody of the desired specificity. This is achieved by fusing lymphocytes to a suitable immortalized fusion partner cell line and selecting fusion product hybridoma cell lines which produce appropriate antibody and grow continuously in culture. The lymphocytes can be obtained from a donor immunized with a core lipopolysaccharide (CLPS) vaccine from a mutant Gram-negative bacterium lacking O-polysaccharide side chains. Suitable organisms include, for example, Salmonella minnesota R595 or E. coli J5. Other suitable organisms can be determined easily by persons skilled in the art. The lymphocytes can be of blood, spleen or lymph node origin and can be obtained in accordance with conventional techniques. A single cell suspension of the lymphocytes containing mononuclear B-lymphocytes can be stimulated in vitro by an antigen and/or transformed by Epstein-Barr viral infection prior to cell fusion.

Immortalization of the immune cells can be achieved by fusion of the cells to an appropriate human, rodent or human/rodent hybrid myeloma, B-lymphoblastoid or other immortalized cell line sufficient to permit continuous hybridoma growth and monoclonal antibody secretion. Suitable cell lines include the mouse myeloma cell line P3X63-Ag8.653 (Gigliotti, F. et al., J. Infectious Diseases 149(1):43 [1984]), the human-mouse myeloma SHM-D33 (Teng. N. et al., PNAS 80:7308 [1983]) and the human lymphoblastoid cell line WI-L2 (Heitzmanna, J. et al., Mol. Biol. Med. 1:235 [1983]).

The fusion process can be carried out in accordance with conventional procedures, such as with polyethylene glycol in calcium- and magnesium-free phosphate buffered saline. Alternatively, the cells can be fused using electric fields or any other acceptable method of cell fusion. Hybrid cells are selected and the number of viable hybrids are increased in accordance with conventional methods. Hybrid selection can be by appropriate toxic drugs, physical methods, immunological methods or any other conventional method that suppresses the growth of unfused tumor cell. Supernatants are harvested to test for immunoglobulin specificity against CLPS. An enzyme linked immunosorption assay (ELISA) in which monoclonal antibodies are detected by their capacity to specifically bind to lipopolysaccharide antigens bound to a solid surface can be used. In order to detect only antigens associated with the core glycolipid region, LPS molecules lacking polysaccharide side chains are used. Preferably, the molecules, which may include LPS from the immunizing organism, are selected from two different Gram-negative microorganisms in order to select the retain cross-reactive mAb. The hybridoma cell culture supernatants are incubated with the CLPS antigens, then the binding of the anti-CLPS antibodies to the CLPS antigens is determined by the subsequent addition of enzyme labeled anti-immunoglobulin antibody. If the hybridoma supernatant contains antibodies to common core glycolipid antigens, the antibody will specifically bind to the antigens bound to the solid surface and be retained on the surface of the microtiter plate containing those antigens but not on the negative control wells. The secretion of anti-CLPS antibodies can be further characterised based on the cross-reactivity of the supernatants against a second CLPS antigen as determined by ELISA analysis. The in vitro assays need not be limited to ELISA but can include RIA or other conventional method of detecting an antigen/antibody interaction.

The selected hybridomas then are cloned to ensure that the antibody cross-reactivity detected in the ELISA screening can be attributed to a monoclonal antibody. Cloning can be accomplished in accordance with any technique known in the art for single cell isolation and culture, including plating cells in limiting dilutions, growth in semi-solid medium or micromanipulation. After clones have been identified and grown to confluency, supernatants from wells containing hybridoma colonies are tested for the presence of antibodies against at least two different common core glycolipid antigens using ELISA as described above. The CLPS antigens can be selected from lipopolysaccharides from any wild type Gram-negative bacterium, lipopolysaccharides from any mutant Gram-negative strain, such as E. coli J5 or S. minnesota R595, the lipid A from any of these bacteria, or detoxified forms of any of these materials. Several clones from each hybridoma having monoclonal antibodies which cross-react with more than one of the LPS antigens are retained. The process of cloning may need to be repeated in order to insure the selection of stable cell lines.

In order to facilitate further analysis of the monoclonal antibody both in vitro and in vivo it is advantageous to have the monoclonal antibody in a purified and concentrated form. One major source of foreign protein contamination is the fetal calf serum normally found in hybridoma growth media. To circumvent this problem, hybridomas which produce monoclonal antibodies of interest can be conditioned to grow in serum-free defined cell culture media by culturing the cells in media containing progressively lower concentrations of fetal calf serum. Cells are selected for their ability to adapt, grow and secrete monoclonal antibodies following sequential passage into lower-serum defined cell culture media. A serum-free medium can include any defined formulation consisting of a mixture of single components sufficient to support hybridoma growth and monoclonal antibody secretion and facilitate monoclonal antibody purification. Typically, these media contain a variety of nutrients and growth supplements, including salts, sugars, vitamins, amino acids, hormones and buffers. The protein additives in the media typically are limited to albumin, insulin, and transferrin.

Concentration of the monoclonal antibodies contained in a serum-free, defined medium can be achieved by passing the cell culture supernatant over an ion exchange resin under conditions which permit binding of the protein constituents to the ion-exchange resin. The proteins then can be eluted by using a buffer of appropriate pH and ionic strength. The eluate contains all the proteins in the culture supernatant but in higher concentrations. The monoclonal antibody in such an eluate typically is 100-fold concentrated relative to the starting material.

Purification of the monoclonal antibodies away from the other protein constituents can be achieved by exploiting the size difference between either IgG or IgM (depending upon the immunoglobulin class of the monoclonal antibodies to be purified) and the other proteins; albumin, transferrin and insulin. This can be achieved by passing the ion-exchange resin eluate over an appropriate molecular size exclusion gel column and selecting the appropriate peak. The monoclonal antibody purified under these conditions typically is quite concentrated and constitutes the majority of the protein in the fraction.

To further define the antigenic specificity of the monoclonal antibodies, additional in vitro testing can be conducted. This testing can include use of an ELISA as described above using LPS or LPS subunits from other mutant or wild-type organisms. For example, the ability of a monoclonal antibody to bind an LPS subunit, such as lipid A, in an ELISA defines the epitope against which the monoclonal antibody is directed as being expressed on the lipid A moiety.

The anti-lipid A monoclonal antibodies obtained can be used in combination with immune globulin products for the prophylactic or therapeutic treatment of diseases of Gram-negative bacterial origin as described below.

As noted above, the monoclonal antibodies obtained can be either IgG or IgM. In one embodiment of this invention, the DNA encoding the monoclonal antibodies can be engineered so as to construct an intra-species or inter-species anti-CLPS monoclonal antibody and/or can be inserted into an alternative procaryotic or eucaryotic expression system. For example, it is known that the specific binding of antigen by antibodies is determined by the structure of the variable regions of both the heavy and light chains of immunoglobulins, while the effector function are determined by the structure of the constant region of the heavy chains. Both the heavy and light chains are encoded by multiple DNA segments. By means of standard recombinant DNA techniques, it is possible to attach any variable region to any heavy chain constant region. For example, chimeric genes containing mouse variable and human constant region domains have been constructed, cloned, and expressed. Such hybrid genes can be used to express monoclonal antibodies of significantly reduced immunogenicity in man, while retaining antigenic specificity. Chimeric monoclonal antibodies could be produced against immunogens which cannot be injected into humans or which normally elicit a weak immune response with limited immunoglobulin production in vivo or in vitro.

Chimeric molecules have been produced within and between species from a variety of novel combinations of immunoglobulin constant and variable regions, as well as from immunoglobulin sequences fused to non-immunoglobulin sequences. The hybrid genes have been introduced into myeloma or lymphoid cells using conventional techniques including transfection, protoplast fusion, and electroporation, although alternatively, the genes can be expressed in nonlymphoid cells such as bacteria or yeast. (Morrison, S.L., 1985, Science 229: 1202-1207 [1985]; Liu, A.Y., et al., Proc. Natl. Acad. Sci USA 84: 3439-3443 [1987]; James et al., J. Immun. Meth. 100: 5 [1987]. Regardless of the source or process from which the monoclonal antibody is obtained, the antibody recognizes and binds to an epitope of the endotoxin from a variety of Gram-negative bacterial species thereby reducing the lethal effects of Gram-negative infections.

### Polyclonal Antibody-Containing Component

Polyclonal antibodies useful in the composition and methods of this invention can comprise purified IgG, IgM, IgA or combinations of classes of the immunoglobulins. IgG immunoglobulins are preferred. Plasma containing normal or high levels of specific polyclonal antibodies can be used. High levels of antibody can be achieved by any one of several methods. Donors can be immunized to obtain plasma containing high titers of antibodies to one or more specific antigens, such as an antigen of P. aeruginosa. Alternatively, it is known that a significant portion of the human population contains plasma which naturally has a high level of antibodies against Gram-negative bacteria. Although the reason for this is unknown, the incidence can be used advantageously by setting up a program of screening donors for high titers of naturally occurring antibodies against one or more antigens of interest. For purposes of this invention, plasma is considered to contain a "high titer" of antibodies to Gram-negative bacteria if the titer of these antibodies is at least 5 times greater than that of normal plasma as determined by in vitro testing. Desirably, the immunoglobulin prepared from such plasma has a titer of antibodies to Gram-negative bacteria greater than 10 times the antibody level in normal plasma pool.

A polyclonal immunoglobulin concentrate can be obtained from blood plasma using procedures known in the art, including cold ethanol precipitation, dialysis, ion exchange adsorption, and concentration by ultrafiltration. Alternative procedures include, but are not limited to, precipitation procedures using polyethylene glycol, polypropylene glycol, inorganic salts (e.g., ammonium sulfate or sodium sulfate), ion-exchange adsorptions with media containing carboxymethyl, diethylaminoethyl, or quaternary aminoethyl functional groups, immunoadsorption, affinity adsorption, isoelectric precipitation, surface adsorption, or gel filtration.

Polyclonal intravenous immunoglobulins produced by incubation at an acidic pH, incubation with trace amounts of pepsin at an acidic pH, incubation with pepsin or plasmin, reduction and alkylation, sulfonation, treatment with B-propiolactone or treatment with hydroxyethyl starch also can be used as the source of the plasma-derived polyclonal immunoglobulin to which the hybridoma-derived monoclonal antibodies are added. Alternatively, polyclonal immunoglobulins also may be formulated at a low pH (e.g., 3.5 - 5.5) in the presence or absence of polyhydroxy compounds such as maltose or sorbitol.

The polyclonal immunoglobulin solution then can be sterilized in accordance with conventional procedures. Then the immunoglobulin can be stabilized and stored in either a liquid or freeze-dried condition. The stable polyclonal immunoglobulins generally have concentrations ranging from about 10 mg/ml to about 200 mg/ml and are suitable for intramuscular, intraperitoneal, and intravenous injection. Undesirable side reactions are avoided by eliminating IgG aggregates, contaminants with vasoactive or coagulant potential, such as PKA or thrombin, and by the significant reduction of other non-IgG proteins, such as IgA and IgE.

The polyclonal immunoglobulin product can be mixed with the monoclonal antibodies prior to or at the time of administration to produce the compositions of this invention. Alternatively, the polyclonal antibody component and the monoclonal antibody component of the compositions of this invention can be administered separately, but within a period of time which enables the polyclonal immunoglobulin and monoclonal antibodies to produce substantially the same therapeutically beneficial result as is obtained if the two components had been mixed together prior to administration. "Composition" thus is used herein to refer to combinations of polyclonal immunoglobulin and monoclonal antibodies, as herein described, which are administered separately or in combination. Compositions comprising polyclonal immunoglobulin and monoclonal antibodies has been shown to be more protective than either component administered alone.

The compositions of the present invention can be administered for the propylactic and/or therapeutic treatment of Gram negative bacterial diseases to patients in need of such treatment. In therapeutic administrations, the compositions are administered to patients already suffering from a disease of Gram-negative bacterial origin, in amounts sufficient to cure or at least partially arrest the infection and accompanying endotoxic shock. An amount adequate to achieve this is referred to as a therapeutically effective dose. Amounts effective for this purpose will vary, depending upon the severity of the infection and the patient's own immune system, but generally are in the range of about 0.25 to about 3 mg monoclonal antibody and about 300 mg to about 1 g polyclonal intravenous immunoglobulins (IGIV) per kg of body weight. Doses of about 0.5 to about 1 mg mAb and about 500 mg IGIV per kg body weight are preferred.

Alternatively, the compositions of this invention can be administered prophylactically to patients judged to be at risk of acquiring a serious Gram-negative bacterial infection. An amount adequate to prevent or, at least significantly lessen the severity of, an infection is referred to as a prophylactically effective dose. Again, amounts effective for this purpose will vary, but generally they are in the range of about 0.1 to about 1 mg monoclonal antibody and about 50 mg to about 500 mg IGIV per kg body weight. Preferred doses are in the range of about 0.25 mg to about 0.5 mg/kg mAb and about 200-400 mg IGIV/kg.

The compositions can be administered as a single administration or as multiple administrations, with the treatment pattern and dose levels being determined by the treating physician to provide quantities of the antibodies sufficient to effectively treat the patient.

The following examples are intended to illustrate the present invention, but they are not to be construed as limiting.

### Example 1

### Production of Human Hybridomas Secreting Human Monoclonal anti-CLPS Antibodies

### a) Fusion

Hybridomas described in this invention can be generated in accordance with any of a variety of techniques known to persons skilled in the art. See, for example, the techniques described in the following references, all of which are essentially equivalent with regard to their potential to produce cell lines secreting anti-endotoxin monoclonal antibodies (James, K. and Bell, G.T., 1987, J. Immunol. Methods 100:5-40. Pollack, M., Raubitschek, A.A., and Larrick, J.W., 1987, J. Clin. Invest. 79: 1421-1430. Teng, N.N.H., Kaplan, H.S., Herber, J.M., et.al. 1985. Proc. Natl. Acad. Sci. USA 82:1790-1794.)

After the cell lines have been generated it is necessary to identify those cell lines producing the desired antibody. This process may begin as soon as the cell line cultures are microscopically observed to be at least partially confluent. A convenient method for detecting specific antibody production is an enzyme linked immunosorbant assay (ELISA) as described below.

In the following procedures, hybridoma cells were generated substantially in accordance with the teachings of Pollack et al., referenced above. The lymphocytes were obtained from a human donor immunized with a CLPS vaccine from S. minnesota R595. The immune cells were fused to cells of the human lymphoblastoid cell line WI-L2-729-HF₂ (WI-L2).

### b) Enzyme-Linked Immunsorption Assay (ELISA)

All the wells of polystyrene 96-well microtitre plates were activated with 0.1 ml 5% glutaraldehyde in distilled water at 37°C for one hour. The wells then were washed ten times with water and emptied. While the plates were incubating with the glutaraldehyde, 5 mg of S. minnesota R595 LPS (List Biological Laboratories, Inc.) were dissolved in 5 ml phosphate buffered saline (PBS) pH 7.4 containing 5 »l triethanolamine (TEA) and 19 mg ethylenediaminetetraacetate (EDTA). One tenth milliliter of this solution then was added to 10 ml of PBS containing 10 »l TEA. Then, 0.1 ml of an LPS-containing solution was added to the wells of the glutaraldehyde-activated plate and incubated for one hour at 37°C. Following the incubation period, the wells were washed 5 times with PBS, emptied, and 0.1 ml of a PBS solution containing 0.1 M glycine and 10 mg/ml bovine serum albumin (BSA) was added to each well to block unreacted binding sites.

After emptying the wells, 0.1 ml of cell-free supernatant was transferred from each hybridoma-containing well of the fusion plates to an individual well of the ELISA microtitre plate and incubated at 37°C for one hour. After washing the plates 10 times with PBS and 0.05% Tween 20, and emptying the wells, 0.1 ml of an appropriately diluted anti-human Ig (Zymed Laboratories, Inc., South San Francisco, Ca.) conjugated to alkaline phosphatase diluted in PB8, 1% BSA, O.05% Tween 20 was added to each well. Following a one hour incubation at 37°C, the ELISA plate was washed seven times with PBS, 0.05% Tween 20, the wells emptied, and 0.1 ml of a solution of 1 mg/ml paranitrophenyl phosphate in 1 M diethanolamine, pH 9.6, was added to each well.

The optical density at 405 nm was determined for each well at 20, 40 and 60 minutes. A hybridoma positive for anti-LPS antibody production was determined by identifying the corresponding ELISA plate well in which the O.D. value was higher than control wells which received all reagent additions except hybridoma supernatants.

Three to four days following the first ELISA testing of hybridoma supernatants, those hybridomas which were positive against S. minnesota R595 LPS were retested in the ELISA system described above, with the variation that LPS from E. coli J5 (List Biological Laboratories, Inc.) was used as the antigen. Those hybridoma antibodies which reacted against LPS from these two antibodies were retained as candidates for monoclonal antibodies which recognize antigens common to the core region of LPS. Among the candidates retained were, for example, two antibodies designated HR78 and HR89. Samples of the hybridomas produced HR78 and HR89 have been deposited with the American Type Culture Collection (ATCC), Rockville, MD, in accordance with the terms of the Budapest Treaty and assigned accession numbers HB9760 and HB9759, respectively.

### c) Cloning

Hybridomas which produce antibodies reactive in the two ELISA tests described above were cloned by limiting dilution. For each hybridoma, 150 viable cells were suspended in 100 ml of RPMI 1640, 20% fetal calf serum (FCS) containing 2.5 x 10⁶ murine thymocytes per ml as feeder cells. Into each well of four 96-well culture plates was pipetted 0.2 ml of this cell suspension and the plates were incubated at 37°C in a humidified chamber. After 10 days, the plates were examined microscopically for growth of hybridoma colonies. As each well originally received approximately 0.3 cells, it was assumed that the hybridoma colonies were derived from a single cell and were, therefore, monoclonal.

Colonies within the well were re-fed by replacing half of the media with fresh media. When the cell colonies were partially confluent, supernatants were tested in ELISA as described above to confirm that the monoclonal antibody had retained cross-reactivity against core glycolipid antigens. Several clones showing strong ELISA activity and good growth were retained from each parental hybridoma.

### d) Growth in Serum-free Media

Four clones of each parental hybridoma were seeded at 10⁵ cells/ml in four 75 cm² tissue culture flasks in defined media. The media used was Hana Biologicals HB104 supplemented with 20% FCS. After four days, the cells were harvested, counted and seeded as before, with the exception that the FCS content was decreased by one-half to 10%. The cells were passaged similarly every three to four days, with the FCS being decreased each time by one-half, provided that the average cell density in the four flasks was at least 4 x 10⁵ cells/ml. If the cells had not reached that minimum density, they were cultured again using the same FCS concentration. If, after three identical passages, the cells were unable to reach a density of 4 x 10⁵ cells/ml, the clone was assumed to be unadaptable to serum-free conditions and discontinued. By evaluating four clones of each parental hybridoma, the chances were increased that at least one clone would grow in serum-free media.

Once a hybridoma had been grown successfully in media containing 1.25% FCS, it was passed in serum-free defined media. After the third passage, cell-free supernatants were sampled and the concentration of human monoclonal antibody determined by quantitative ELISA. The clone from each hybridoma with the highest antibody production was selected for scale-up, antibody purification and evaluation.

### Example 2

### Purification of Human Monoclonal Anti-CLPS Antibodies

### a) Isotype Determination

In order to determine amounts of monoclonal antibodies contained in various samples, such as culture supernatants, an ELISA is performed. The assay is constructed in such as a way as to be specific for either human IgG or human IgM in order to accommodate monoclonal antibodies of either isotype. Thus, once a monoclonal antibody is selected for its capacity to recognize common core LPS epitopes and for the capacity of the hybridoma to grow in serum-free media, the isotype of the monoclonal antibody is determined.

To determine the isotype of the monoclonal antibody, the ELISA of Example 1, section b, was used with a slight modification. Specifically, the monoclonal antibody was allowed to bind to the antigen in duplicate wells. Then, alkaline phosphatase conjugated anti-human IgG or anti-human IgM was added to each well. The rest of the ELISA was run in accordance with the procedure described above, and the isotype of the monoclonal antibody was determined by reactivity with the relevant alkaline phosphatase conjugate.

### b) Quantitative ELISA

Following the determination of the isotype, a quantitative ELISA was performed to measure the concentration of IgG or IgM in the monoclonal antibody-containing samples. For IgM antibodies, each well of a polystyrene 96-well microtitre plate was filled with 0.1 ml of a solution containing 10 »g/ml anti-human IgM. The plates were incubated for one hour at 37°C, then washed five times with PBS and the wells emptied. The plates then were blocked by the addition of 0.1 ml per well of a PBS, 1% bovine serum albumin solution and incubated at 37°C for one hour. Monoclonal antibody-containing samples were added in a volume of 0.1 ml to duplicate wells. A standard curve then was generated using an IgM source, specifically, human serum containing a known amount of IgM. The amount of IgM in the serum was determined using a commercially available test system such as radial immunodiffusion or laser nephelometry. Preferably, more than one method of measurement is used in order to obtain confirmation of the IgM content of the standard. The quantitative ELISA is most accurate when the IgM concentration is in the range of 0.1 to 1.0 »g/ml. Therefore, the IgM standard was diluted with PBS such that at least four sets of the duplicate wells fell within that concentration range. Test samples anticipated to contain greater than 1 »g/ml of monoclonal antibody IgM were appropriately diluted to bring the test sample concentration within the standard curve concentration range.

To measure the concentration of IgG antibodies, the same procedure is followed, with the exception that the wells of the microtitre plate were filled with a solution containing anti-human IgG and human serum containing a known amount of IgG is used to generate the standard curve.

### c) Monoclonal Antibody Purification

Hybridoma cells were grown in defined media and six 3 liter spinner flasks were seeded at 10⁵ cells/ml. Because the media was to be used as a source material for the purification of monoclonal antibody, it had to be selected to meet two requirements. First, because the monoclonal antibody was to be tested for its anti-bacterial activity, no antimicrobial agent could be present in the monoclonal antibody to be tested. Therefore, antibiotics, such as penicillin or streptomycin, were not used. Secondly, because the monoclonal antibodies are against endotoxin, the supernatants had to be pyrogen free. Therefore, all glassware and other materials to come into contact with the mAb had to be depyrogenated and all media had to be prepared pyrogen-free, including the use of pyrogen free water. Pyrogen testing was carried out using a commercial limulus amoebocyte lysate (LAL) test. Media prior to culturing normally contain less than 0.25 endotoxin units (EU)/ml and culture supernatants were normally less than 2.0 EU/ml.

After three to five days of culture, supernatants were harvested by first pumping them past a sterile pyrogen-free glass fibre filter to remove cells and cell debris. The clarified supernatant then was passed through a Millipak® 50 0.22 »M filter (Millipore) to remove particulate material. The 18 L supernatant pool then was passed over a strong anion-exchanger ZetaPrep® (Cuno Inc.) 100 cartridge at a flow rate of 1 L/hour. After the culture supernatant was loaded on the filter, the filter was washed with 0.15 N NaCl, 0.05 M Tris buffer pH 7.5 until excess protein was removed as detected by an in-line UV detector and determining absorbance at 280 nM. The proteins were eluted using a solution of 0.5 N NaCl, 0.05 M Tris buffer pH 7.5, and the first 50 ml of the protein-containing peak was collected. This peak was applied to a 5 cm X 50 cm column of Sephacryl S-300® (Pharmacia) pre-equilibrated with 0.1 M NaCl, 0.05 M Tris pH 7.5 and running at 120 ml/hour. The first protein peak as detected by A280 containing IgM was collected in a volume of 60 ml.

The purified mAb was tested for total protein, IgM by quantitative ELISA, endotoxin by LAL and was analyzed by polyacrylamide gel electrophoresis (PAGE). Typically, the protein content of the sample was between 100 »g/ml and 200 »g/ml. The mAb constituted about one half of the total protein of the sample; the other half consisted primarily of albumin as demonstrated by PAGE. Such preparations normally contain 1-3 EU/ml. Monoclonal antibody prepared in this manner is suitable for further evaluation in vitro and in vivo.

### Example 3

### In vitro Determination of Antigenic Specificity

Samples of the mAb purified in Example 2 were tested for in vitro reactivity to various lipopolysaccharides by ELISA. The mAb was first diluted to a concentration of 10 »g/ml. The antigens against which the mAb were tested included the LPS from S. minnesota R595 and E. coli J5 as described in Example 1, section b. The lipid A from R595 LPS and various LPS extracted from wild-type Gram-negative bacteria also were used.

The mAb which demonstrated binding to the lipid A antigen were retained for further evaluation with polyclonal immunoglobulin in vivo for their capacity to protect animals from death by endotoxic shock.

### Example 4

### The in vivo Determination of Antibody Activity

To develop an animal model most accurately reflecting the human clinical setting of Gram-negative bacterial infection, the lethal effects of both the viable organisms and the endotoxin had to be taken into account. In order to do this, various antibody preparations were tested for their capacity to protect test animals against lethal challenge doses containing both viable bacteria and endotoxin purified from the same organism.

### a) Human polyclonal IgG

The experiment described below was conducted in accordance with the following general protocol: Outbred Swiss Webster mice were first given the polyclonal antibody preparation as a 0.3 ml intravenous injection. One and one half hours later, the mice were given an intraperitoneal injection of 15 mg galactosamine hydrochloride dissolved in O.2 ml sterile pyrogen-free saline to increase their sensitivity to endotoxin. One half hour later, the animals were challenged with lethal doses of viable E. coli 07Kl organisms grown to mid-log phase, or endotoxin, or a combination of organisms plus endotoxin.

To determine the efficacy of human polyclonal intravenous immunoglobulin in this model, an experiment was conducted wherein 116 female Swiss Webster mice weighing 16-18 grams each were divided into four groups of 24 mice each and two groups of 10 mice each. All the mice received an intravenous (I.V.) injection of either sterile pyrogen-free saline or 10 mg IGIV (intravenous human immuno-globulin). IGIV was prepared by weighing out an appropriate amount of powder from a lyophilized sample of Gammagard®, (Baxter Travenol Laboratories, Inc.) and dissolving it in pyrogen-free water for injection. The protein concentration was adjusted to 50 mg per ml using the A280 reading on a Gilford spectrophotometer. IGIV was diluted to 33 1/3 mg/ml in sterile pyrogen-free saline. Mice received 0.3 ml IV or 10 mg IGIV. Endotoxin sensitivity was increased using galactosamine hydrochloride, and all challenge doses were administered intraperitoneally.

After 48 hours, the number of surviving mice was as follows: in unprotected mice (those receiving saline), 62.5% of the mice survived in the group challenged with organisms alone: 50% of the mice survived in the group challenged with endotoxin alone, and 41.6% of the mice survived challenge with the combination of organisms plus endotoxin (Table 1). In mice protected with 10 mg IGIV, about 83% of the mice survived challenge with organisms alone, 60% of the mice survived in the group challenged with endotoxin alone and 37.5% of the mice survived challenge with the combination of organisms and endotoxin (Table 1). Thus, IGIV protected mice against bacterial infection, however, it failed to provide similar protection against a lethal organism challenge containing added endotoxin.

**Table 1**

| Effect of Gammagard Pretreatment on Female CFW Mice Challenged with E. coli 07Kl Endotoxin and/or Organisms | | | | | | |
|---|---|---|---|---|---|---|
| Protection | Survivors/Total No. of challenge organisms | | | | | |
| | Challenge | 0 | 2.3x10¹ | 2.3x10² | 2.3x10³ | % Survival |
| Saline | 10 ng E. coli 07Kl LPS | 5/10 | --- | --- | --- | 50.0 |
| | E. coli | --- | 6/8 | 7/8 | 2/8 | 62.5 |
| | E. coli plus 10 ng LPS | --- | 2/8 | 4/8 | 4/8 | 41.6 |
| 10 mg IGIV | 10 ng E. coli 07Kl LPS | 6/10 | --- | --- | --- | 60.0 |
| | E. coli 07Kl | --- | 8/8 | 7/8 | 5/8 | 83.3 |
| | E. coli plus 10 ng LPS | --- | 4/8 | 4/8 | 1/8 | 37.5 |

### b) Human anti-endotoxin monoclonal antibody

To determine the efficacy of human anti-endotoxin monoclonal antibody, the following animal model was used. The anti-endotoxin monoclonal antibodies tested were HR78 and HR89 (see Example 1(b)). Antibody HR78 is of the IgM class and has kappa light chains. It reacts in vitro with LPS from S. minnesota R595, E. coli J5 and lipid A from E. coli from S. minnesota R595. It reacted only weakly in vitro with wild type LPS from S. minnesota and E. coli 0111:B4 (Table 2). However, in vitro activity against wild type lipopolysaccharides may be inhibited due to the presence of polysaccharide side chains. Antibody HR89 is of the IgM class and has kappa light chains. In reacts in vitro with LPS from S. minnesota R595 and E. coli J5 and lipid A from S. minnesota and E. coli (Table 2). Similarly to HR78, it does not react strongly in vitro with the LPS isolated from wild-type S. minnesota or E. coli.

Ninety age and sex-matched Swiss Webster mice were divided into three groups (groups A, B, and C) of 30 mice each. Group A received 10 mg IGIV in 0.3 ml sterile saline. Group B received 10 »g of the test anti-endotoxin mAb in 0.3 ml sterile saline. Group C received a 0.3 ml I.V. injection consisting of 1O mg IGIV and 10 »g mAb. One and a half hours after the I.V. injection, all the mice received 15 mg galactosamine intraperitoneally. One half hour later, the following intraperitoneal challenges were administered: ten mice in each group received 10 E. coli 07Kl organisms plus 10 nanograms E. coli 07Kl lipopolysaccharide; 10 mice received 100 organisms plus 10 nanograms LPS and 10 mice received 1000 organisms plus 10 nanograms LPS.

After 48 hours, rates of survival in Groups A, B, and C were determined. A survival rate in Group C which is significantly higher than the survival rate in Groups A and B would indicate that the mAb is effective. Significance was determined by p values generated using a 2 x 2 contingency test. Monoclonal antibodies HR78 and HR89 showed significant protective activity.

In vivo. the combination of HR78 plus IGIV protected mice against E. coli 07Kl plus endotoxin better than either the antibody or IGIV alone (see Table 3, Experiment 1). Similarly in vivo, the combination of HR89 plus IGIV protected mice against E. coli 07Kl organisms plus endotoxin better than either HR89 or IGIV alone (Table 3, Experiment 2).

**Table 2**

| LPS Antigen | A₄₀₅ Test mAB | |
|---|---|---|
| | HR78 | HR89 |
| S. minnesota wild type | 0.105 | 0.204 |
| S. minnesota R595 | 0.509 | 0.529 |
| S. minnesota Lipid A | 0.401 | 0.666 |
| E. coli 0111:B4 | 0.008 | 0.264 |
| E. coli J5 | 0.483 | 0.658 |
| E. coli Lipid A | 0.618 | 0.762 |

**Table 3**

| Combined Effect of Core LPS mAb and GAMMAGARD on CFW Mice Challenged with E. coli 07Kl Organisms plus Endotoxin | | | | | |
|---|---|---|---|---|---|
| GROUPS | Survivors 10 | | | Total Survivors/30 | 2p |
| | 10 ng E. coli 07K1 Endotoxin plus Organisms | | | | |
| | 1.3 x 10¹ | 1.3 x 10² | 1.3 x 1O³ | | |
| Experiment 1 | | | | | |
| A) 10 mg GAMMAGARD (lot number 860415AGll) | 4 | 5 | 5 | 14 | |
| B) 10»g HR 78 | 3 | 5 | 1 | 9 | |
| C) lOmg GAMMAGARD + lO»g HR78 | 9 | 8 | 8 | 25 | .001¹ <.003² |

| Experiment 2 | | | | | |
|---|---|---|---|---|---|
| A) lO mg GAMMAGARD (lot number 860415AGll) | 3 | 4 | 3 | 10 | |
| B) lO»g HR89 | 4 | 3 | 5 | 12 | |
| C) lO»g GAMMAGARD + lO»g HR89 | 9 | 8 | 5 | 22 | <.003¹ .009² |

| | | | | | |
|---|---|---|---|---|---|
| 1) Group C vs Group A | | | | | |
| 2) Group C vs Group B | | | | | |

## Claims (Claims for the following Contracting State(s): AT, BE, CH, DE, FR, GB, IT, LI, NL, SE)

1. A composition comprising polyclonal immunoglobulins containing polyclonal antibodies against antigens of Gram-negative bacteria and monoclonal antibodies capable of binding with and neutralizing an antigen common to the endotoxin region of a variety of Gram-negative bacterial lipopolysaccharides.

2. A composition in accordance with claim 1, wherein the monoclonal antibodies exhibit IgG or IgM isotype.

3. A composition in accordance with claim 1, wherein the polyclonal immunoglobulins contain antibodies that recognize O-antigens specific for a variety of Gram-negative organisms.

4. A composition in accordance with claim 1, wherein the monoclonal antibodies are obtained from a hybridoma obtained by fusing immune lymphocytes to an immortalized cell line fusion partner and selecting a fusion product hybridoma cell line.

5. A composition in accordance with claim 4, wherein the immune lymphocytes have been infected with Epstein-Barr virus prior to fusion.

6. A composition in accordance with claim 1, wherein the monoclonal antibodies are cross-reactive to at least two different core lipopolysaccharides from two Gram-negative bacteria genera.

7. A composition in accordance with claim 1, wherein the monclonal antibodies are mammalian.

8. A composition in accordance with claim 7, wherein the monoclonal antibodies are human.

9. A composition in accordance with claim 1 wherein the monoclonal antibody is an intra-species chimera.

10. A composition in accordance with claim 1, wherein the monoclonal antibody is an inter-species chimera.

11. A composition in accordance with claim 1 wherein the polyclonal immunoglobulin has a titer of antibodies to one or more antigens of Gram-negative bacteria in a range of at least 5 times that found in normal plasma as determined by an ELISA.

12. A composition in accordance with claim 1, wherein the polyclonal immunoglobulin has a titer of antibodies to one or more antigens of Gram-negative bacteria in a range of at least 10 times that found in normal plasma as determined by an ELISA.

13. A composition in accordance with claim 11 or 12, wherein the plasma was obtained from human donors who had been vaccinated with a Gram-negative bacterial antigen vaccine.

14. A composition in accordance with claim 11 or 12, wherein the plasma was obtained from human donors who possess naturally high antibody titer to at least one Gram-negative organism.

15. A composition according to any one of claims 1 - 14 wherein the said polyclonal antibodies and the said monoclonal antibodies are presented separately from one another and as a kit of parts.

16. A prophylactic or therapeutic composition for use in treating a patient at risk of acquiring, or suffering from a disease of Gram-negative bacterial origin and including a composition according to any one of Claims 1 to 14.

17. Use of a composition according to any one of Claims 1 to 14 in the manufacture of a prophylactic or therapeutic composition for use in the treatment of a patient suffering from a disease of Gram-negative bacterial origin.

18. Use of polyclonal antibodies against antigens of Gram-negative bacteria in the manufacture of a medicament for the treatment or prophylaxis of diseases of Gram-negative bacterial origin in a patient, the treatment or prophylaxis comprising also the administration to the patient of monoclonal antibodies capable of binding to the endotoxin region of a variety of Gram-negative bacterial lipopolysaccharides.

19. Use of monoclonal antibodies capable of binding to the endotoxin region of a variety of Gram-negative bacterial lipopolysaccharides in the manufacture of a medicament for the treatment or prophylaxis of diseases of Gram-negative bacterial origin in a patient, the treatment or prophylaxis comprising also the administration to the patient of polyclonal antibodies against antigens of Gram-negative bacteria.

## Claims (Claims for the following Contracting State(s): ES)

1. A process for preparing a composition comprising admixing polyclonal antibodies against antigens of Gram-negative bacteria and monoclonal antibodies capable of binding with and neutralizing an antigen common to the endotoxin region of a variety of Gram-negative bacterial lipopolysaccharides.

2. A process in accordance with claim 1, wherein the monoclonal antibodies exhibit IgG or IgM isotype.

3. A process in accordance with claim 1, wherein the polyclonal immunoglobulins contain antibodies that recognize O-antigens specific for a variety of Gram-negative organisms.

4. A process in accordance with claim 1, wherein the monoclonal antibodies are obtained from a hybridoma obtained by fusing immune lymphocytes to an immortalized cell line fusion partner and selecting a fusion product hybridoma cell line.

5. A process in accordance with claim 4, wherein the immune lymphocytes have been infected with Epstein-Barr virus prior to fusion.

6. A process in accordance with claim 1, wherein the monoclonal antibodies are cross-reactive to at least two different core lipopolysaccharides from two Gram-negative bacteria genera.

7. A process in accordance with claim 1, wherein the monoclonal antibodies are mammalian.

8. A process in accordance with claim 7, wherein the monoclonal antibodies are human.

9. A process in accordance with claim 1 wherein the monoclonal antibody is an intra-species chimera.

10. A process in accordance with claim 1, wherein the monoclonal antibody is an inter-species chimera.

11. A process in accordance with claim 1, wherein the polyclonal immunoglobulin has a titer of antibodies to one or more antigens of Gram-negative bacteria in a range of at least 5 times that found in normal plasma as determined by an ELISA.

12. A process in accordance with claim 1, wherein the polyclonal immunoglobulin has a titer of antibodies to one or more antigens of Gram-negative bacteria in a range of at least 10 times that found in normal plasma as determined by an ELISA.

13. A process in accordance with claim 11 or 12, wherein the plasma was obtained from human donors who had been vaccinated with a Gram-negative bacterial antigen vaccine.

14. A process in accordance with claim 11 or 12, wherein the plasma was obtained from human donors who possess naturally high antibody titer to at least one Gram-negative organism.

15. A process according to any one of claims 1 to 14, wherein the said polyclonal antibodies and the said monoclonal antibodies are presented separately from one another and as a kit of parts.

16. Use of polyclonal antibodies against antigens of Gram-negative bacteria in the manufacture of a medicament for the treatment or prophylaxis of diseases of Gram-negative bacterial origin in a patient, the treatment or prophylaxis comprising also the administration to the patient of monoclonal antibodies capable of binding to the endotoxin region of a variety of Gram-negative bacterial lipopolysaccharides.

17. Use of monoclonal antibodies capable of binding to the endotoxin region of a variety of Gram-negative bacterial lipopolysaccharides in the manufacture of a medicament for the treatment or prophylaxis of diseases of Gram-negative bacterial origin in a patient, the treatment or prophylaxis comprising also the administration to the patient of polyclonal antibodies against antigens of Gram-negative bacteria.

## Patentansprüche (Patentansprüche für folgende(n) Vertragsstaat(en): AT, BE, CH, DE, FR, GB, IT, LI, NL, SE)

1. Zusammensetzung, die polyklonale Immunglobuline aufweist, die polyklonale Antikörper gegen Antigene von gramnegativen Bakterien und monoklonale Antikörper enthält, die fähig sind, an ein Antigen, das dem Endotoxinbereich einer Vielzahl von gramnegativen bakteriellen Lipopolysacchariden gemeinsam ist, zu binden und es zu neutralisieren.

2. Zusammensetzung nach Anspruch 1, wobei die monoklonalen Antikörper IgG- oder IgM-Isotypie zeigen.

3. Zusammensetzung nach Anspruch 1, wobei die polyklonalen Immunglobuline Antikörper enthalten, die O-Antigene erkennen, die für eine Vielzahl von gramnegativen Organismen spezifisch sind.

4. Zusammensetzung nach Anspruch 1, wobei die monoklonalen Antikörper von einem Hybridom erhalten sind, das erhalten ist durch Fusion von Immunlymphozyten mit einem immortalisierten Zellinien-Fusionspartner und Auswählen einer Fusionsprodukt-Hybridomzellinle.

5. Zusammensetzung nach Anspruch 4, wobei die Immunlymphozyten vor der Fusion mit Epstein-Barr-Virus infiziert worden sind.

6. Zusammensetzung nach Anspruch 1, wobei die monoklonalen Antikörper mit wenigstens zwei verschiedenen Kern-Lipopolysacchariden von zwei gramnegativen Bakterienarten kreuzreaktionsfähig sind.

7. Zusammensetzung nach Anspruch 1, wobei die monoklonalen Antikörper Säugerantikörper sind.

8. Zusammensetzung nach Anspruch 7, wobei die mnoklonalen Antikörper Humanantikörper sind.

9. Zusammensetzung nach Anspruch 1, wobei der monoklonale Antikörper eine Intraspezies-Chimäre ist.

10. Zusammensetzung nach Anspruch 1, wobei der monoklonale Antikörper eine Interspezies-Chimäre ist.

11. Zusammensetzung nach Anspruch 1, wobei das polyklonale Immunglobulin einen Antikörpertiter für ein oder mehr Antigene von gramnegativen Bakterien in einem Bereich von wenigstens dem Fünffachen desjenigen hat, der sich in normalem Plasma findet, gemäß der Bestimmung durch einen ELISA.

12. Zusammensetzung nach Anspruch 1, wobei das polyklonale Immunglobulin einen Antikörpertiter für ein oder mehr Antigene von gramnegativen Bakterien in einem Bereich von wenigstens dem Zehnfachen desjenigen hat, der sich in normalem Plasma findet, gemäß der Bestimmung durch einen ELISA.

13. Zusammensetzung nach Anspruch 11 oder 12, wobei das Plasma von menschlichen Spendern erhalten wurde, die mit einem gramnegativen bakteriellen Antigen-Impfstoff geimpft worden waren.

14. Zusammensetzung nach Anspruch 11 oder 12, wobei das Plasma von menschlichen Spendern erhalten wurde, die einen natürlich hohen Antikörpertiter für wenigstens einen gramnegativen Organismus besitzen.

15. Zusammensetzung nach einem der Ansprüche 1 bis 14, wobei die polyklonalen Antikörper und die monoklonalen Antikörper separat voneinander und als ein Set von Teilen präsentiert werden.

16. Prophylaktische oder therapeutische Zusammensetzung zur Verwendung bei der Behandlung eines Patienten, bei dem das Risiko des Erwerbs einer Erkrankung eines gramnegativen bakteriellen Ursprungs besteht oder der daran leidet, wobei diese Zusammensetzung eine Zusammensetzuung nach einem der Ansprüche 1 bis 14 aufweist.

17. Verwendung einer Zusammensetzung nach einem der Ansprüche 1 bis 14 bei der Herstellung einer prophylaktischen oder therapeutischen Zusammensetzung zur Verwendung bei der Behandlung eines Patienten, der an einer Erkrankung eines gramnegativen bakteriellen Ursprungs leidet.

18. Verwendung von polyklonalen Antikörpern gegen Antigene von gramnegativen Bakterien bei der Herstellung eines Medikaments zur Behandlung oder Prophylaxe von Erkrankungen eines gramnegativen bakteriellen Ursprungs bei einem Patienten, wobei die Behandlung oder Prophylaxe auch die Verabreichung von monoklonalen Antikörpern an den Patienten umfaßt, die fähig sind, an den Endotoxinbereich einer Vielzahl von gramnegativen bakteriellen Lipopolysacchariden zu binden.

19. Verwendung von monoklonalen Antikörpern, die fähig sind, an den Endotoxinbereich einer Vielzahl von gramnegativen bakteriellen Lipopolysacchariden zu binden, bei der Herstellung eines Medikaments zur Behandlung oder Prophylaxe von Erkrankungen eines gramnegativen bakteriellen Ursprungs bei einem Patienten, wobei die Behandlung oder Prophylaxe auch die Verabreichung von polyklonalen Antikörpern gegen Antigene von gramnegativen Bakterien an den Patienten aufweist.

## Patentansprüche (Patentansprüche für folgende(n) Vertragsstaat(en): ES)

1. Verfahren zum Herstellen einer Zusammensetzung, das folgendes aufweist Beimischen von polyklonalen Antikörpern gegen Antigene von gramnegativen Bakterien, und von monoklonalen Antikörpern, die fähig sind, an ein Antigen, das dem Endotoxinbereich einer Vielzahl von gramnegativen bakteriellen Lipopolysacchariden gemeinsam ist, zu binden und es zu neutralisieren.

2. Verfahren nach Anspruch 1, wobei die monoklonalen Antikörper IgG- oder IgM-Isotypie zeigen.

3. Verfahren nach Anspruch 1, wobei die polyklonalen Immunglobuline Antikörper enthalten, die O-Antigene erkennen, die für eine Vielzahl von gramnegativen Organismen spezifisch sind.

4. Verfahren nach Anspruch 1, wobei die monoklonalen Antikörper von einem Hybridom erhalten sind, das erhalten ist durch Fusion von Immunlymphozyten mit einem immortalisierten Zellinien-Fusionspartner und Auswahlen einer Fusionsprodukt-Hybridomzellinie.

5. Verfahren nach Anspruch 4, wobei die Immunlymphozyten vor der Fusion mit Epstein-Barr-Virus infiziert worden sind.

6. Verfahren nach Anspruch 1, wobei die monoklonalen Antikörper mit wenigstens zwei verschiedenen Kern-Lipopolysacchariden von zwei gramnegativen Bakterienarten kreuzreaktionsfähig sind.

7. Verfahren nach Anspruch 1, wobei die monoklonalen Antikörper Säugerantikörper sind.

8. Verfahren nach Anspruch 7, wobei die monoklonalen Antikörper Humanantikörper sind.

9. Verfahren nach Anspruch 1, wobei der monoklonale Antikörper eine Intraspezies-Chimäre ist.

10. Verfahren nach Anspruch 1, wobei der monoklonale Antikörper eine Interspezies-Chimäre ist.

11. Verfahren nach Anspruch 1, wobei das polyklonale Immunglobulin einen Antikörpertiter für ein oder mehr Antigene von gramnegativen Bakterien in einem Bereich von wenigstens dem Fünffachen desjenigen hat, der sich in normalem Plasma findet, gemäß der Bestimmung durch einen ELISA.

12. Verfahren nach Anspruch 1, wobei das polyklonale Immunglobulin einen Antikörpertiter für ein oder mehr Antigene von gramnegativen Bakterien in einem Bereich von wenigstens dem Zehnfachen desjenigen hat, der sich in normalem Plasma findet, gemäß der Bestimmung durch einen ELISA.

13. Verfahren nach Anspruch 11 oder 12, wobei das Plasma von menschlichen Spendern erhalten wurde, die mit einem gramnegativen bakteriellen Antigen-Impfstoff geimpft worden waren.

14. Verfahren nach Anspruch 11 oder 12, wobei das Plasma von menschlichen Spendern erhalten wurde, die einen natürlich hohen Antikörpertiter für wenigstens einen gramnegativen Organismus besitzen.

15. Verfahren nach einem der Ansprüche 1 bis 14, wobei die polyklonalen Antikörper und die monoklonalen Antikörper separat voneinander und als ein Set von Teilen dargeboten werden.

16. Verwendung von polyklonalen Antikörpern gegen Antigene von gramnegativen Bakterien bei der Herstellung eines Medikaments zur Behandlung oder Prophylaxe von Erkrankungen eines gramnegativen bakteriellen Ursprungs bei einem Patienten, wobei die Behandlung oder Prophylaxe auch die Verabreichung von monoklonalen Antikörpern an den Patienten aufweist, die fähig sind, an den Endotoxinbereich einer Vielzahl von gramnegativen bakteriellen Lipopolysacchariden zu binden.

17. Verwendung von monoklonalen Antikörpern, die fähig sind, an den Endotoxinbereich einer Vielzahl von gramnegativen bakteriellen Lipopolysacchariden zu binden, bei der Herstellung eines Medikaments zur Behandlung oder Prophylaxe von Erkrankungen eines gramnegativen bakteriellen Ursprungs bei einem Patienten, wobei die Behandlung oder Prophylaxe auch die Verabreichung von polyklonalen Antikörpern gegen Antigene von gramnegativen Bakterien an den Patienten aufweist.

## Revendications (Revendications pour l'(les) Etat(s) contractant(s) suivant(s): AT, BE, CH, DE, FR, GB, IT, LI, NL, SE)

1. Composition comprenant des immunoglobulines polyclonales contenant des anticorps polyclonaux contre les antigènes de bactéries Gram-négatives et des anti-corps monoclonaux capables de se fixer à un antigène commun à la région endotoxine de toute une gamme de lipopolysaccharides de bactéries Gram-négatives et les neutraliser.

2. Composition selon la revendication 1, dans laquelle les anticorps monoclonaux présentent l'isotype IgG ou IgM.

3. Composition selon la revendication 1, dans laquelle les immunoglobulines polyclonales contiennent des anticorps qui reconnaissent les antigènes O spécifiques de toute une gamme d'organismes Gram-négatifs.

4. Composition selon la revendication 1, dans laquelle on obtient les anticorps monoclonaux à partir d'un hybridome que l'on a obtenu par fusion de lymphocytes immuns sur un partenaire de fusion constitué d'une lignée cellulaire immortalisée, et en sélectionnant une lignée cellulaire d'hybridome obtenue par fusion.

5. Composition selon la revendication 4, dans laquelle les lymphocytes immuns ont été infectés par le virus Epstein-Barr avant la fusion.

6. Composition selon la revendication 1, dans laquelle les anticorps monoclonaux présentent une réactivité croisée avec au moins deux lipopolysaccharides de coeur différents provenant de deux espèces de bactéries Gram-négatives.

7. Composition selon la revendication 1, dans laquelle les anticorps monoclonaux proviennent de mammifères.

8. Composition selon la revendication 7, dans laquelle les anticorps monoclonaux sont humains.

9. Composition selon la revendication 1, dans laquelle l'anticorps monoclonal est une chimère intra-espèces.

10. Composition selon la revendication 1, dans laquelle l'anticorps monoclonal est une chimère inter-espèces.

11. Composition selon la revendication 1, dans laquelle l'immunoglobuline polyclonale a un titre d'anticorps contre un ou plusieurs antigènes de bactéries Gram-négatives, compris dans un intervalle au moins 5 fois égal à celui que l'on trouve dans le plasma normal, tel que déterminé par une analyse ELISA.

12. Composition selon la revendication 1, dans laquelle l'immunoglobuline polyclonale a un titre d'anticorps contre un ou plusieurs antigènes de bactéries Gram-négatives, compris dans un intervalle au moins 10 fois égal à celui que l'on trouve dans le plasma normal, tel que déterminé par une analyse ELISA.

13. Composition selon la revendication 11 ou 12, dans laquelle le plasma est obtenu à partir de donneurs humains qui ont été vaccinés avec un vaccin à antigène bactérien Gram-négatif.

14. Composition selon la revendication 11 ou 12, dans laquelle le plasma est obtenu de donneurs humains qui présentent un titre d'anticorps naturellement élevé contre au moins un organisme Gram-négatif.

15. Composition selon l'une quelconque des revendications 1 à 14, dans laquelle les anticorps polyclonaux et les anticorps monoclonaux sont présentés séparément les uns des autres, dans une trousse.

16. Composition prophylactique ou thérapeutique, destinée à être utilisée pour le traitement d'un patient soumis au risque d'acquérir ou de présenter une maladie d'origine bactérienne Gram-négative, et comprenant une composition selon l'une quelconque des revendications 1 à 14.

17. Utilisation d'une composition selon l'une quelconque des revendications 1 à 14 pour préparer une composition prophylactique ou thérapeutique destinée à être utilisée dans le traitement d'un patient souffrant d'une maladie d'origine bactérienne Gram-négative.

18. Utilisation d'anticorps polyclonaux contre les antigènes de bactéries Gram-négatives lors de la préparation d'un médicament destiné au traitement ou à la prophylaxie de maladies d'origine bactérienne Gram-négative chez un patient, le traitement ou la prophylaxie comprenant aussi l'administration, au patient, d'anticorps monoclonaux pouvant se fixer à la région endotoxine de toute une gamme de lipopolysaccharides de bactéries Gram-négatives.

19. Utilisation d'anticorps monoclonaux pouvant se fixer à la région endotoxine de toute une gamme de lipopolysaccharides de bactéries Gram-négatives lors de la préparation d'un médicament destiné au traitement ou à la prophylaxie de maladies d'origine bactérienne Gram-négative chez un patient, le traitement ou la prophylaxie comprenant aussi l'administration, au patient, d'anticorps monoclonaux contre les antigènes de bactéries Gram-négatives.

## Revendications (Revendications pour l'(les) Etat(s) contractant(s) suivant(s): ES)

1. Procédé pour préparer une composition, qui consiste à mélanger des anticorps polyclonaux contre les antigènes de bactéries Gram-négatives et des anticorps monoclonaux pouvant se fixer à un antigène commun à la région endotoxine de toute une gamme de lipopolysaccharides de bactéries Gram-négatives, et les neutraliser.

2. Procédé selon la revendication 1, dans lequel les anticorps monoclonaux présentent l'isotype IgG ou IgM.

3. Procédé selon la revendication 1, dans lequel les immunoglobulines polyclonales contiennent des anticorps qui reconnaissent les antigènes O spécifiques de toute une gamme d'organismes Gram-négatives.

4. Procédé selon la revendication 1, dans lequel on obtient les anticorps monoclonaux à partir d'un hybridome que l'on a obtenu par fusion de lymphocytes immuns sur un partenaire de fusion constitué d'une lignée cellulaire immortalisée, et en sélectionnant une lignée cellulaire d'hybridome obtenue par fusion.

5. Procédé selon la revendication 4, dans lequel les lymphocytes immuns ont été infectés par le virus Epstein-Barr avant la fusion.

6. Procédé selon la revendication 1, dans lequel les anticorps monoclonaux présentent une réactivité croisée avec au moins deux lipopolysaccharides de coeur différents provenant de deux espèces de bactéries Gram-négatives.

7. Procédé selon la revendication 1, dans lequel les anticorps monoclonaux proviennent de mammifères.

8. Procédé selon la revendication 7, dans lequel les anticorps monoclonaux sont humains.

9. Procédé selon la revendication 1, dans lequel l'anticorps monoclonal est une chimère intra-espèces.

10. Procédé selon la revendication 1, dans lequel l'anticorps monoclonal est une chimère inter-espèces.

11. Procédé selon la revendication 1, dans lequel l'immunoglobuline polyclonale a un titre d'anticorps contre un ou plusieurs antigènes de bactéries Gram-négatives, compris dans un intervalle au moins 5 fois égal à celui que l'on trouve dans le plasma normal, tel que déterminé par une analyse ELISA.

12. Procédé selon la revendication 1, dans lequel l'immunoglobuline polyclonale a un titre d'anticorps contre un ou plusieurs antigènes de bactéries Gram-négatives, compris dans un intervalle au moins 10 fois égal à celui que l'on trouve dans le plasma normal, tel que déterminé par une analyse ELISA.

13. Procédé selon la revendication 11 ou 12, dans lequel le plasma est obtenu à partir de donneurs humains qui ont été vaccinés avec un vaccin à antigène bactérien Gram-négatif.

14. Procédé selon la revendication 11 ou 12, dans lequel le plasma est obtenu de donneurs humains qui présentent un titre d'anticorps naturellement élevé contre au moins un organisme Gram-négatif.

15. Procédé selon l'une quelconque des revendications 1 à 14, dans lequel les anticorps polyclonaux et les anticorps monoclonaux sont présentés séparément les uns des autres, sous forme d'une trousse.

16. Utilisation d'anticorps polyclonaux contre les antigènes de bactéries Gram-négatives lors de la préparation d'un médicament destiné au traitement ou à la prophylaxie de maladies d'origine bactérienne Gram-négative chez un patient, le traitement ou la prophylaxie comprenant aussi l'administration, au patient, d'anticorps monoclonaux pouvant se fixer à la région endotoxine de toute une gamme de lipopolysaccharides de bactéries Gram-négatives.

17. Utilisation d'anticorps monoclonaux pouvant se fixer à la région endotoxine de toute une gamme de lipopolysaccharides de bactéries Gram-négatives lors de la préparation d'un médicament destiné au traitement ou à la prophylaxie de maladies d'origine bactérienne Gram-négative chez un patient, le traitement ou la prophylaxie comprenant aussi l'administration, au patient, d'anticorps monoclonaux contre les antigènes de bactéries Gram-négatives.
